(19) **Europäisches Patentamt**
**European Patent Office**
**Office européen des brevets**

(11) **EP 4 606 350 A1**

(12) **EUROPEAN PATENT APPLICATION**
published in accordance with Art. 153(4) EPC

(43) Date of publication:
**27.08.2025  Bulletin 2025/35**

(21) Application number: **23880097.3**

(22) Date of filing: **10.10.2023**

(51) International Patent Classification (IPC):
***A61F 2/16*** *(2006.01)*

(52) Cooperative Patent Classification (CPC):
**A61F 2/16**

(86) International application number:
**PCT/KR2023/015524**

(87) International publication number:
**WO 2024/085518 (25.04.2024 Gazette 2024/17)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **18.10.2022  KR 20220133757**

(71) Applicant: **Losec Co., Ltd.**
**Uiwang-si, Gyeonggi-do 16006 (KR)**

(72) Inventor: **PARK, Kyoungjin**
**Uiwang-si Gyeonggi-do 16006 (KR)**

(74) Representative: **Jilderda, Anne Ayolt**
**LIOC Patents & Trademarks**
**Zwaanstraat 31 L**
**5651 CA Eindhoven (NL)**

(54) **CONNECTION SUPPORT FOR INTRAOCULAR LENS**

(57)  An intraocular lens according to one aspect of the present invention comprises:
an optic part (100) and a haptic part (200),
wherein the haptic part (200) includes a connecting portion (220) connected to the optic part (100) and at least three ring-shaped loops (210) extending from the connecting portion (220).

Fig. 1

EP 4 606 350 A1

## Description

### TECHNICAL FIELD

One aspect of the present invention relates to a connecting support for intraocular lenses, which has not previously existed. The invention specifically concerns a connecting support for intraocular lenses capable of efficiently transmitting forces received by the capsular bag to the intraocular lens while simultaneously ensuring that the intraocular lens is properly positioned within the capsular bag.

### BACKGROUND ART

Korean Registered Patent No. 10-0843454, by the inventor, discloses a support structure for intraocular lenses. Unlike conventional capsular tension rings used during cataract surgery, which maintain the circular contour of the capsular bag but often result in adhesion between the anterior and posterior capsules, thereby compromising the lens thickness adjustment of the intraocular lens, this support structure effectively resolves this issue. It efficiently transmits relaxation and contraction forces from the ciliary body (zonule) to the intraocular lens within the capsular bag, thereby providing superior accommodative functionality similar to the natural crystalline lens.

Additionally, Korean Registered Patent No. 10-1718075 discloses connecting means for intraocular lenses. The connecting means serve to efficiently transfer forces from the support structure to the intraocular lens by connecting the intraocular lens to the support structure.

By mediating through the connecting means, it is possible to reduce the size of the support structure, thereby reducing the incision length during cataract surgery. Furthermore, the connecting means enable the intraocular lens to be firmly connected within the support structure, thus effectively transferring forces.

Although the aforementioned support structure and connecting means introduced new concepts previously unavailable, not all intraocular lenses have been manufactured in forms compatible with such supports or connecting means. In other words, compatibility issues arise when using support structures and connecting means with intraocular lenses, highlighting the need for standardized supports and connecting means that can universally interface with all intraocular lenses.

### [Prior Art Documents]

Prior Art Document 1: Korean Patent No. 10-0843454

### DISCLOSURE

### Technical Problem

One aspect of the present invention aims to provide a connecting support having a standardized inner surface compatible with intraocular lenses, enabling stable positioning of any intraocular lens within the capsular bag, regardless of the type of intraocular lens.

Another aspect of the present invention provides a connecting support for intraocular lenses, integrating both a support structure and connecting means. This integrated structure effectively transfers external forces delivered to the capsular bag to the intraocular lens while simultaneously ensuring stable positioning of the intraocular lens against its inner surface.

### Technical Solution

The connecting support according to one aspect of the present invention comprises:
A ring-shaped structure provided internally along the equator of the capsular bag for intraocular lens implantation, wherein, in a cross-section along the visual axis direction, the wall of the structure has inner and outer surfaces convex toward the capsular bag, and the wall includes:

a front section positioned anteriorly along the visual axis direction;

a rear section extending from the front section and positioned posteriorly relative to the equator; and

fixation means provided on the inner surface of the wall for securing an end portion of the haptic part.

Preferably, the fixation means is a bent portion extending from the rear section and bent inward toward the interior of the eye.

Further, the inner surface of the rear section may be a curved surface around the intersection point with the bent portion, while the inner surface of the bent portion may be a planar surface.

Moreover, at the intersection point between the inner surfaces of the rear section and the bent portion, the angle formed between a tangent line on the inner surface of the rear section and the inner surface of the bent portion may range from 45° to 110°.

Additionally, around the intersection between the inner surfaces of the rear section and the bent portion, both inner surfaces may be curved surfaces.

Furthermore, at the intersection of these curved inner surfaces, the angle formed between tangent lines on the surfaces may range from 45° to 110°.

Also, the connecting support preferably has a partially open section spanning an angle ranging from 1° to 30° measured from the center.

Further, it is preferable that the overall diameter of the connecting support varies from 9.3 mm to 12 mm depending on the eye size, while the inner diameter remains constant.

Moreover, the maximum thickness (x, mm) between the outer and inner surfaces of the connecting support preferably satisfies $0.35 \leq x$.

Additionally, it is preferable that the maximum thickness of the connecting support is inversely proportional to its hardness.

Furthermore, the maximum thickness (x, mm) and hardness (y, Shore hardness) of the connecting support preferably satisfy the following formula (Formula 1):

$$-196.08x^3 + 523.43x^2 - 503.5x + 204 \leq y \leq 196.08x^3 + 523.43x^2 - 503.5x + 207$$

(where $0.35 \leq x$)

Another aspect of the invention, an intraocular lens assembly, comprises:

a connecting support for intraocular lenses inserted internally along the equator of a capsular bag, wherein, in a cross-section along the visual axis direction, the wall of the structure has inner and outer surfaces convex toward the capsular bag, including:

a front section positioned anteriorly along the visual axis direction;

a rear section extending from the front section and positioned posteriorly relative to the equator;

fixation means provided on an inner surface of the wall for securing an end portion of the haptic part; and

an intraocular lens including an optic part and a haptic part, wherein the haptic part contacts the inner surface of the connecting support, and the end portion of the haptic part is seated within the bent portion.

Preferably, the optic part of the intraocular lens moves anteriorly or posteriorly according to forces transmitted from the ciliary body to the intraocular lens capsular bag.

**Advantage Effects**

As described above, the connecting support for intraocular lenses according to one aspect of the present invention integrates two primary functions: efficiently transferring forces from the capsular bag to the intraocular lens, and securely positioning and fixing the haptic part of the intraocular lens without the need for additional connecting means. Consequently, it facilitates simple and stable fixation of the intraocular lens at a designated position (i.e., the center) during intraocular lens implantation surgery.

Furthermore, the connecting support according to one aspect of the invention moves the intraocular lens anteriorly through a bent portion that supports the optic part of the intraocular lens when forces that deform the lens are transmitted. Thus, in addition to the excellent accommodative properties similar to the natural crystalline lens provided by previously proposed supports, the present invention can achieve higher visual correction effects.

Additionally, the connecting support according to one aspect of the present invention enables vision correction to the desired diopter post-surgery. Specifically, when an intraocular lens of a predetermined diopter is inserted, it is consistently positioned in the intended location, thus preventing variations in corrected vision among individuals and allowing precise vision correction as intended.

Therefore, the connecting support according to one aspect of the present invention provides accommodative properties even when using standard intraocular lenses without separate accommodative functionalities.

**BRIEF DESCRIPTION OF DRAWINGS**

FIG. 1 is a perspective view of an intraocular lens connecting support according to a first embodiment of the present invention.

FIG. 2 is a cross-sectional view taken along line A-A of FIG. 1.

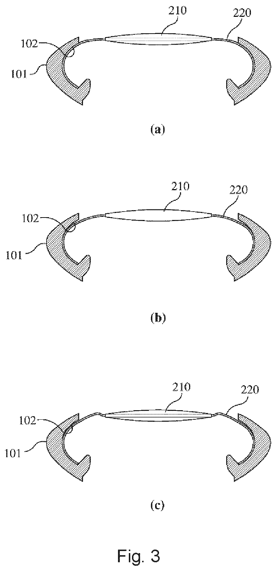

FIG. 3 is a graph illustrating the relationship between the hardness and maximum thickness of the intraocular lens connecting support.

FIG. 4 is a cross-sectional view illustrating cases where an intraocular lens of a first type is coupled in different orientations to the intraocular lens connecting support according to the first embodiment of the present invention.

FIG. 5 is a plan view of various types of intraocular lenses that can be used with the connecting support according to the present invention.

FIG. 6 is an explanatory diagram illustrating the operation of the intraocular lens when viewing distant and near objects, respectively, according to one embodiment of the present invention.

DETAILED DESCRIPTION OF THE EMBODIMENTS

Hereinafter, exemplary embodiments of the present invention will be described in detail with reference to the accompanying drawings. The present invention may be embodied in various different forms and is not limited to the embodiments described herein.

Before describing the present invention in detail, it should be understood that the terminology used herein is for the purpose of describing particular embodiments only and is not intended to limit the scope of the invention, which is defined solely by the appended claims. All technical and scientific terms used herein have the same meanings as commonly understood by one of ordinary skill in the technical field, unless otherwise indicated.

Throughout the specification and claims, the terms "comprise," "comprises," and "comprising" mean the inclusion of the mentioned items, steps, or groups of items and steps, and do not exclude other items, steps, or groups of items or steps.

Meanwhile, various embodiments of the present invention can be combined with any other embodiments unless explicitly indicated otherwise. Specifically, any features indicated as preferred or advantageous may be combined with other features similarly indicated as preferred or advantageous.

**In** the drawings, the dimensions such as width, length, and thickness of components may be exaggerated for convenience. Descriptions throughout the drawings are presented from an observer's perspective. When one component is described as being "above/below" or "on/under" another component, this includes cases where there is another component positioned between them, as well as cases where one component is directly above or below the other.

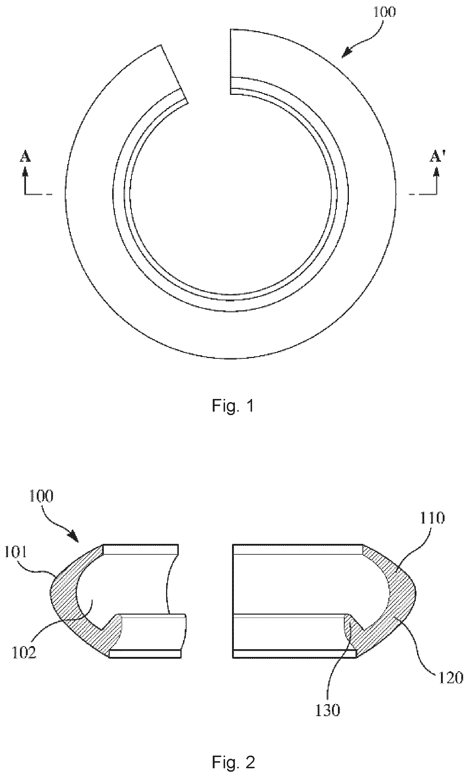

FIG. 1 is a plan view of a connecting support for intraocular lenses according to one embodiment of the present invention, and FIG. 2 is a cross-sectional view taken along line A-A of FIG. 1.

**In** the present invention, the connecting support (100) maintains the shape of the capsular bag similarly to previously proposed supports, resolves the issue of adhesion between the anterior and posterior capsules of the capsular bag, and efficiently transmits the relaxation and contraction of the ciliary body (zonule) to the intraocular lens, thus enabling the intraocular lens to provide vision with depth perception similar to a natural crystalline lens.

Furthermore, the connecting support (100) of the present invention has a unique structure with functionalities not present in conventional supports. Specifically, the connecting support features a standardized inner surface, allowing it to universally accommodate intraocular lenses regardless of the shape of their haptic parts, as long as their sizes match this standardized dimension. This standardized inner surface eliminates the need to manufacture intraocular lenses in various sizes, facilitating standardization to a single size.

Additionally, the connecting support (100) includes fixation means for the haptic part, ensuring precise central positioning of the intraocular lens within the eye. The fixation means also enable anterior movement of the intraocular lens in response to forces transmitted from the ciliary body, thereby providing excellent visual acuity when viewing distant objects.

The connecting support (100) is inserted into the capsular bag of a human eye during cataract surgery before the intraocular lens, with its outer surface fixed by contacting the equator of the capsular bag. Subsequently, the intraocular lens is inserted, and its haptic part is seated on the inner surface of the connecting support.

The connecting support (100) is configured as an open or closed ring-shaped structure. As shown in FIG. 2, in a cross-section taken along a virtual plane in the lens's visual axis direction (Y direction), the structure has walls with inner and outer surfaces that are convex outwardly. In cross-section, these walls comprise a front section (110), a rear section (120), and fixation means (130), delineated by the equator of the support structure.

The front section (110) is positioned anteriorly relative to an equatorial line, defined as a line connecting both convex apexes of the connecting support (100). The rear section (120) extends from the front section and is positioned posteriorly relative to the equatorial line connecting the convex apexes of the connecting support. The fixation means (130) is provided in a region of the wall and functions to secure an end portion of the haptic part of the intraocular lens.

A preferred example of the fixation means (130) is a bent portion. This bent portion extends from the rear section and is bent inward toward the interior direction. The end portion of the haptic part of the intraocular lens may be seated either at the intersection point of the inner surfaces of the rear section and the bent portion or on the inner surface of the bent portion itself.

The intersection point of the inner surface of the rear section (120) and the inner surface of the bent portion (130) forms a region suitable for seating the end portion of the haptic part. Even if the length of the haptic part is relatively long, it can extend beyond this intersection and be seated on the inner surface of the bent portion.

Specifically, if the inner surface of the rear section (120) around its intersection with the bent portion (130) is a curved surface and the inner surface of the bent portion is planar, the angle formed in cross-section at the intersection point between the tangent line of the inner surface of the rear section and the planar inner surface of the bent portion may range from 45° to 110°, preferably 60° to 100°.

Alternatively, if both the inner surface of the rear section (120) and the inner surface of the bent portion (130) around their intersection point are curved surfaces, the angle formed in cross-section at the intersection point between the tangent lines of these curved surfaces may range from 45° to 110°, preferably 60° to 90°.

If the angle exceeds the specified range, the haptic part of the intraocular lens may not seat properly on the inner surface of the bent portion or at the intersection point between the inner surfaces of the rear section and the bent portion. Conversely, if the angle is below this range, longer haptic parts might pass beyond the intersection point and fail to seat on the inner surface of the bent portion.

Another example of fixation means is a protrusion formed on the inner surface of the connecting support wall. Unlike the bent portion, this protrusion extends and projects from the inner surface of the rear section along the ring shape, but its function of seating the haptic part of the intraocular lens is the same as that of the bent portion.

Yet another example of fixation means includes additionally forming a seating groove on the previously described bent portion. By adding such a seating groove, the end portion of the haptic part can be positioned more securely.

To facilitate insertion during surgery, the connecting support (100) preferably includes a partially open section. This open section preferably spans an angle ranging from 1° to 30° measured from the center. If the opening is less than 1°, insertion through a minimally incised surgical opening may be difficult, while an opening greater than 30° may reduce the connecting support's ability to stably support the intraocular lens.

The overall diameter of the connecting support may range from 9.3 mm to 12 mm depending on eye size. However, even though the overall diameter may vary, the inner diameter-defined as the distance between opposite inner surfaces of the connecting support-is preferably constant, for example, 8.6 mm. This standardization allows for uniform sizing of the intraocular lenses used with the connecting support. Thus, when using this connecting support, all intraocular lenses can be manufactured to a standardized size based on the connecting support's inner diameter, and lenses of various diopters can be produced as needed within this standardized dimension.

In this case, it is preferable that the thickness (x, mm) between the inner and outer surfaces of a connecting support having a larger overall diameter satisfies $0.35 \leq x$. If the thickness is less than 0.35 mm, the length of the haptic part of intraocular lenses typically ranging from 12 mm to 14 mm in diameter may become insufficient to seat securely on the previously described bent portion.

Additionally, the maximum thickness (x, mm) is preferably inversely proportional to the hardness (y, Shore hardness) of the connecting support. For example, it is preferable to satisfy the following Formula 1:

Formula 1:

$$-196.08x^3 + 523.43x^2 - 503.5x + 204 \leq y \leq 196.08x^3 + 523.43x^2 - 503.5x + 207$$

(where $0.35 \leq x$)

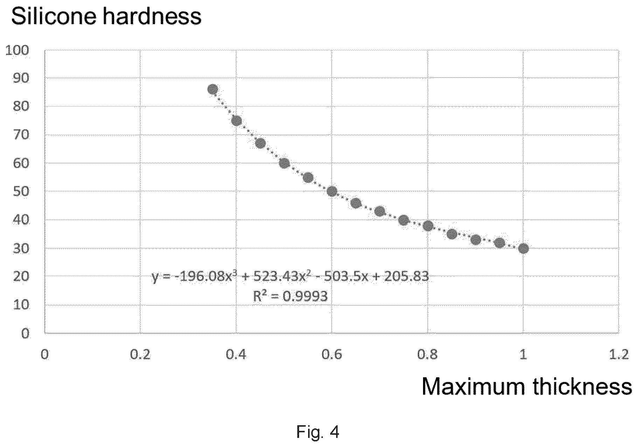

A graph illustrating the relationship between hardness and maximum thickness according to Formula 1 is shown in FIG. 4. Maintaining this inverse proportionality between maximum thickness and hardness ensures that as the thickness increases (leading to an increase in size), the hardness decreases, thus maintaining flexibility. This flexibility facilitates ease of insertion during surgery and reduces the size of the incision required.

The inner surface (102) is preferably smooth, allowing the haptic part of the intraocular lens to glide easily. The bent portion is configured so that the end portion of the haptic part seats securely without displacement, ensuring stable fixation.

The outer surface (101) contacts the inner surface of the capsular bag, making contact at two or more points. To effectively transfer forces delivered to the capsular bag, it is preferable that at least 1/2 to the entire cross-sectional length of the outer surface (101) contacts the capsular bag. Moreover, at least half of the contacting region should include the area around the equator where the ciliary body (zonule) is attached, thereby efficiently transmitting the force from the ciliary body.

Table 1 illustrates an embodiment showing the size, thickness, and hardness of the connecting support. According to this, although the outer diameter of the connecting support varies, the inner diameter remains consistently maintained. It also shows that the connecting support is designed such that its maximum thickness is inversely proportional to the Shore hardness of the silicone.

[Table 1]

| Outer Diameter of Connecting Support | Maximum Thickness of Connecting Support (mm) | Inner Diameter of Connecting Support (mm) | Shore Hardness of Connecting Support |
|---|---|---|---|
| 9.3 | 0.35 | 8.6 | 86 |
| 9.4 | 0.4 | 8.6 | 75 |
| 9.5 | 0.45 | 8.6 | 67 |
| 9.6 | 0.5 | 8.6 | 60 |
| 9.7 | 0.55 | 8.6 | 55 |
| 9.8 | 0.6 | 8.6 | 50 |
| 9.9 | 0.65 | 8.6 | 46 |
| 10 | 0.7 | 8.6 | 43 |
| 10.1 | 0.75 | 8.6 | 40 |
| 10.2 | 0.8 | 8.6 | 38 |
| 10.3 | 0.85 | 8.6 | 35 |
| 10.4 | 0.9 | 8.6 | 33 |
| 10.5 | 0.95 | 8.6 | 32 |
| 10.6 | 1 | 8.6 | 30 |

Meanwhile, the outer diameter of the connecting support described above may be larger or smaller than the described embodiments; for example, it can range from 11.75 mm to 12 mm, in which case the Shore hardness can be 30 or less.

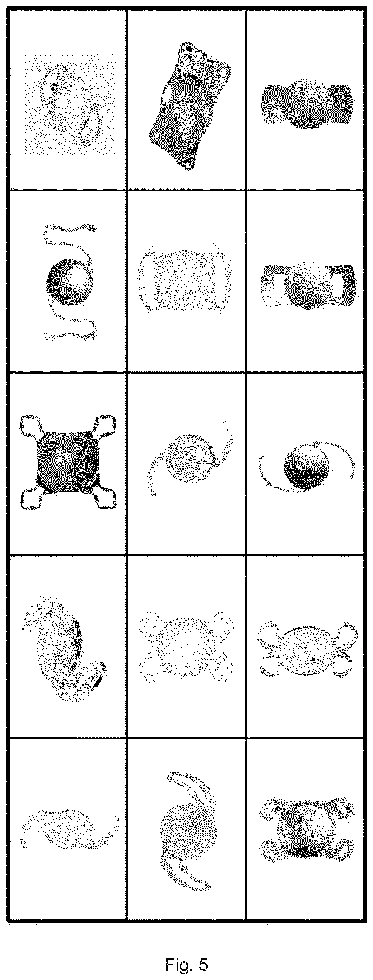

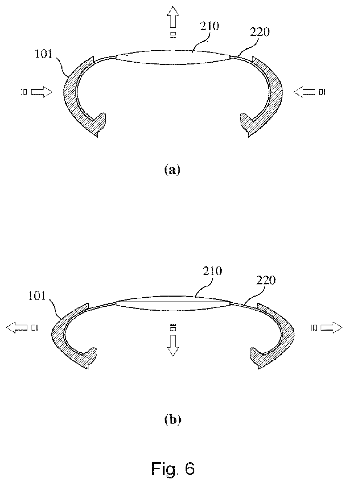

FIG. 5 is a plan view illustrating various shapes of intraocular lenses that can be used with the connecting support according to the present invention, and FIG. 6 is a cross-sectional view showing how the haptic part of the intraocular lens seats within the connecting support.

Accordingly, the variously shaped intraocular lenses (200) shown in FIG. 5 generally consist of an optic part (210) and a haptic part (220). The optic part (210) of the intraocular lens (200) is the lens portion inserted inside the capsular bag, typically having a convex lens shape (although it may have a concave lens shape in some cases of severe myopia).

The haptic part (220) extends from the optic part (210) and serves as a leg portion designed to position the optic part (210) inside the capsular bag, and can be manufactured in various shapes. For instance, it may consist of three radial legs or four legs arranged in a square formation. Additionally, the haptic part (220) can be arranged either in the same plane as the optic part (210) or connected at an angle relative to the optic part. While the shape of the haptic part is not limited in the present invention, its total length should be greater than the inner diameter to ensure that the end portion of the haptic part can be securely seated in the bent portion.

The combined length of the optic part and the haptic part of commercially available intraocular lenses ranges from 12 mm to 14 mm. Commercial intraocular lenses are inserted into the capsular bag initially in a folded state following insertion of a capsular tension ring. As they unfold, the legs forming the haptic part elastically contact and secure against the inner surface of the capsular bag.

When inserting such intraocular lenses directly into the capsular bag, accurate centering of the intraocular lens is often not achieved, and the lens does not effectively receive the forces from the ciliary body (zonule).

However, when using the connecting support according to the present invention, because the maximum thickness of the connecting support satisfies $0.35 \leq x \leq 1$, the intraocular lens cannot fully extend the legs of the haptic part (220) due to spatial constraints within the connecting support. Instead, the legs become flexed and extend until they contact the angled bent portion (130) of the connecting support.

Thus, as shown in FIG. 6, the legs of the haptic part extend while contacting the inner surface of the connecting support until their ends seat in the bent portion. Therefore, the intraocular lens (200), with its haptic part (220) extending along and contacting the inner surface of the connecting support, can effectively receive forces transmitted through the connecting support. Additionally, because both ends of the haptic part seat in the bent portion, centering of the lens becomes straightforward and reliable.

FIG. 6(a) illustrates an intraocular lens with a flat haptic part, while FIGS. 6(b) and 6(c) show intraocular lenses with angled haptic parts in inserted states.

Accordingly, when the haptic part (220) is flat, it bends while contacting the inner surface of the connecting support, with its end seated either on the inner surface of the bent portion (130) or at the intersection of the bent portion (130) and the rear section (120).

This demonstrates that the connecting support according to the present invention can accommodate intraocular lenses with angled haptic parts regardless of their orientation. Particularly, in the configuration shown in FIG. 6(b), where the haptic part angles forward and then bends backward, it strongly facilitates anterior movement of the optic part (210).

FIG. 7 is an explanatory diagram illustrating the operation of the intraocular lens when viewing distant and near objects, respectively, according to an embodiment of the present invention.

As described above, by making the total length of the intraocular lens (200) greater than the inner diameter of the connecting support and forming a bent portion at the ends of the connecting support (100), the optic part of the intraocular lens (200) can be positioned anteriorly relative to the equatorial line.

Thus, since the optic part (210) is positioned anteriorly relative to the equatorial line, the forces transmitted from the capsular bag to the connecting support cause the haptic part (220) to move, thereby enabling anterior movement of the optic part (210) when viewing distant objects. This anterior movement maximizes the visual correction effect.

Although the thickness of the intraocular lens (IOL) itself is typically very thin, making it difficult to significantly alter curvature through transmitted forces, moving the lens forward effectively achieves a higher visual correction effect.

Conversely, when viewing near objects, the optic part (210) moves posteriorly, reversing the mechanism involved in anterior optic movement.

The features, structures, and effects described in each of the above embodiments may be combined or modified by those skilled in the art to create additional embodiments. Therefore, such combinations and modifications should be considered within the scope of the present invention.

**[Description of Reference Numerals]**

100:    Connecting support

101:    Outer surface

102:    Inner surface

110:    Front section

120:    Rear section

130:    Fixation means (bent portion)

200:    Intraocular lens

210:    Optic part

220:     Haptic part

**Claims**

1.  An intraocular lens connecting support comprising a ring-shaped structure provided internally along the equator of a capsular bag, wherein, in a cross-section along the visual axis direction, the wall has inner and outer surfaces convex toward the capsular bag, the wall comprising:

    - a front section positioned anteriorly along the visual axis direction;
    - a rear section extending from the front section and positioned posteriorly relative to the equator; and
    - fixation means provided on an inner surface of the wall for securing an end portion of the haptic part.

2.  The intraocular lens connecting support according to claim 1, wherein the fixation means is a bent portion extending from the rear section and bent inward toward the interior of the eye.

3.  The intraocular lens connecting support according to claim 1, wherein the inner surface of the rear section is a curved surface around the intersection with the bent portion, and the inner surface of the bent portion is a planar surface.

4.  The intraocular lens connecting support according to claim 1, wherein at the intersection of the inner surfaces of the rear section and bent portion, the angle formed between a tangent to the curved surface of the rear section and the planar surface of the bent portion is between 45° and 110°.

5.  The intraocular lens connecting support according to claim 1, wherein, around the intersection of the inner surfaces of the rear section and bent portion, both inner surfaces are curved.

6.  The intraocular lens connecting support according to claim 1, wherein at the intersection of the curved inner surfaces of the rear section and bent portion, the angle formed between tangent lines of these surfaces is between 45° and 110°.

7.  The intraocular lens connecting support according to claim 1, wherein the support has a partially open section spanning an angle of 1° to 30° measured from the center.

8.  The intraocular lens connecting support according to claim 1, wherein the overall diameter varies from 9.3 mm to 12 mm depending on eye size, while the inner diameter remains constant.

9.  The intraocular lens connecting support according to claim 1, wherein the maximum thickness ($x$, in mm) between the outer and inner surfaces satisfies: $0.35 \leq x$.

10. The intraocular lens connecting support according to claim 1, wherein the maximum thickness is inversely proportional to the hardness of the support.

11. The intraocular lens connecting support according to claim 1, wherein the maximum thickness ($x$, mm) and hardness ($y$, Shore hardness) satisfy:

    $$-196.08x^3 + 523.43x^2 - 503.5x + 204 \leq y \leq 196.08x^3 + 523.43x^2 - 503.5x + 207$$

    (where $0.35 \leq x$).

12. An intraocular lens assembly comprising:

    - an intraocular lens connecting support provided internally along the equator of a capsular bag, the support having inner and outer surfaces convex toward the capsular bag in a cross-section along the visual axis direction, the wall including:

        - a front section positioned anteriorly;
        - a rear section extending from the front section posteriorly relative to the equator;
        - fixation means provided on an inner surface of the wall for securing an end portion of the haptic part; and

- an intraocular lens comprising:

- an optic part; and
- a haptic part, wherein the haptic part contacts the inner surface of the connecting support, and the end portion of the haptic part is seated within the bent portion.

13. The intraocular lens assembly according to claim 12, wherein the optic part of the intraocular lens moves anteriorly or posteriorly according to forces transmitted from the ciliary body to the intraocular lens capsular bag.

100

A

A'

Fig. 1

100

101

110

102

120

130

Fig. 2

Fig. 3

Silicone hardness

$y = -196.08x^3 + 523.43x^2 - 503.5x + 205.83$
$R^2 = 0.9993$

Maximum thickness

Fig. 4

Fig. 5

(a)

(b)

Fig. 6

## INTERNATIONAL SEARCH REPORT

| | International application No. |
|---|---|
| | **PCT/KR2023/015524** |

**A. CLASSIFICATION OF SUBJECT MATTER**

**A61F 2/16**(2006.01)i

According to International Patent Classification (IPC) or to both national classification and IPC

**B. FIELDS SEARCHED**

Minimum documentation searched (classification system followed by classification symbols)

A61F 2/16(2006.01); A61F 2/14(2006.01); A61F 9/007(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 안구(eyeball), 캡슐러백(capsular bag), 안구내렌즈(intraocular lens), 햅틱 (haptic), 지지(support), 고정(fixation), 볼록(convex), 절곡(bending), 직경(diameter), 두께(thickness)

**C. DOCUMENTS CONSIDERED TO BE RELEVANT**

| Category* | Citation of document, with indication, where appropriate, of the relevant passages | Relevant to claim No. |
|---|---|---|
| DX | KR 10-1718075 B1 (LOSEC CO., LTD.) 04 April 2017 (2017-04-04)<br>See claims 1-2; paragraphs [0044]-[0045], [0050]-[0057] and [0065]; and figures 5-9. | 1,7,12 |
| DY | | 9,13 |
| DA | | 2-6,8,10-11 |
| Y | JP 2019-535362 A (OMEGA OPHTHALMICS LLC) 12 December 2019 (2019-12-12)<br>See paragraph [0214]. | 9 |
| Y | JP 2003-524503 A (ADVANCED MEDICAL OPTICS, INC.) 19 August 2003 (2003-08-19)<br>See paragraph [0021]; and figure 2. | 13 |
| A | US 2013-0304206 A1 (PALLIKARIS, I. et al.) 14 November 2013 (2013-11-14)<br>See entire document. | 1-13 |

☑ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **02 February 2024** | **02 February 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT

| | International application No. |
|---|---|
| | **PCT/KR2023/015524** |

### C.    DOCUMENTS CONSIDERED TO BE RELEVANT

| Category* | Citation of document, with indication, where appropriate, of the relevant passages | Relevant to claim No. |
|---|---|---|
| DA | KR 10-0843454 B1 (PARK, Kyung Jin) 03 July 2008 (2008-07-03)<br>See entire document. | 1-13 |

Form PCT/ISA/210 (second sheet) (July 2022)

16

## INTERNATIONAL SEARCH REPORT
### Information on patent family members

International application No.

**PCT/KR2023/015524**

| Patent document cited in search report | | | Publication date (day/month/year) | Patent family member(s) | | | Publication date (day/month/year) |
|---|---|---|---|---|---|---|---|
| KR | 10-1718075 | B1 | 04 April 2017 | CN | 107847313 | A | 27 March 2018 |
| | | | | CN | 107847313 | B | 01 December 2020 |
| | | | | EP | 3305250 | A1 | 11 April 2018 |
| | | | | EP | 3305250 | B1 | 20 July 2022 |
| | | | | ES | 2928394 | T3 | 17 November 2022 |
| | | | | JP | 2018-519981 | A | 26 July 2018 |
| | | | | JP | 2019-130376 | A | 08 August 2019 |
| | | | | JP | 6595102 | B2 | 23 October 2019 |
| | | | | JP | 6821736 | B2 | 27 January 2021 |
| | | | | US | 10758339 | B2 | 01 September 2020 |
| | | | | US | 2018-0147049 | A1 | 31 May 2018 |
| | | | | WO | 2016-195143 | A1 | 08 December 2016 |
| JP | 2019-535362 | A | 12 December 2019 | CA | 3040281 | A1 | 26 April 2018 |
| | | | | CA | 3040281 | C | 13 April 2021 |
| | | | | CA | 3109979 | A1 | 26 April 2018 |
| | | | | CA | 3109979 | C | 17 October 2023 |
| | | | | EP | 3528747 | A1 | 28 August 2019 |
| | | | | EP | 3528747 | B1 | 15 September 2021 |
| | | | | EP | 3954326 | A1 | 16 February 2022 |
| | | | | JP | 2020-103906 | A | 09 July 2020 |
| | | | | JP | 6661836 | B2 | 11 March 2020 |
| | | | | JP | 6918158 | B2 | 11 August 2021 |
| | | | | US | 10111746 | B2 | 30 October 2018 |
| | | | | US | 10898315 | B2 | 26 January 2021 |
| | | | | US | 11654016 | B2 | 23 May 2023 |
| | | | | US | 2018-0110613 | A1 | 26 April 2018 |
| | | | | US | 2019-0076239 | A1 | 14 March 2019 |
| | | | | US | 2021-0275293 | A1 | 09 September 2021 |
| | | | | WO | 2018-075932 | A1 | 26 April 2018 |
| JP | 2003-524503 | A | 19 August 2003 | AU | 4538501 | A | 12 September 2001 |
| | | | | BR | 0108909 | A | 24 December 2002 |
| | | | | CA | 2401795 | A1 | 07 September 2001 |
| | | | | CA | 2401795 | C | 09 September 2008 |
| | | | | EP | 1272128 | A2 | 08 January 2003 |
| | | | | JP | 4892156 | B2 | 07 March 2012 |
| | | | | US | 6797004 | B1 | 28 September 2004 |
| | | | | WO | 01-64136 | A2 | 07 September 2001 |
| | | | | WO | 01-64136 | A3 | 25 July 2002 |
| US | 2013-0304206 | A1 | 14 November 2013 | WO | 2013-168141 | A1 | 14 November 2013 |
| KR | 10-0843454 | B1 | 03 July 2008 | AU | 2007-348383 | A1 | 12 September 2008 |
| | | | | BR | PI0715007 | A2 | 28 May 2013 |
| | | | | CA | 2646831 | A1 | 12 September 2008 |
| | | | | CA | 2646831 | C | 29 July 2014 |
| | | | | CN | 101511308 | A | 19 August 2009 |
| | | | | CN | 101511308 | B | 04 April 2012 |
| | | | | EA | 021172 | B1 | 30 April 2015 |
| | | | | EA | 200900385 | A1 | 28 August 2009 |
| | | | | EP | 2117465 | A1 | 18 November 2009 |
| | | | | EP | 2117465 | B1 | 19 July 2017 |
| | | | | ES | 2645530 | T3 | 05 December 2017 |

Form PCT/ISA/210 (patent family annex) (July 2022)

**INTERNATIONAL SEARCH REPORT**
Information on patent family members

International application No.

**PCT/KR2023/015524**

| Patent document cited in search report | Publication date (day/month/year) | Patent family member(s) | | Publication date (day/month/year) |
|---|---|---|---|---|
| | | IL | 196580 A | 18 November 2009 |
| | | IL | 196580 B | 31 March 2015 |
| | | JP | 2010-502358 A | 28 January 2010 |
| | | JP | 2012-130794 A | 12 July 2012 |
| | | JP | 5628235 B2 | 19 November 2014 |
| | | MX | 2009002971 A | 12 August 2009 |
| | | MY | 150396 A | 15 January 2014 |
| | | NZ | 571252 A | 31 May 2013 |
| | | US | 2009-0306774 A1 | 10 December 2009 |
| | | US | 8852275 B2 | 07 October 2014 |
| | | WO | 2008-108523 A1 | 12 September 2008 |
| | | ZA | 200807906 B | 24 February 2010 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 100843454
- KR 101718075